(19) Europäisches Patentamt
European Patent Office
Office européen des brevets

(11) Publication number: 0 147 926 B1

(12) EUROPEAN PATENT SPECIFICATION

(45) Date of publication of patent specification: 03.01.90

(51) Int. Cl.[5]: A 61 K 31/28, C 07 F 15/00

(21) Application number: 84307112.7

(22) Date of filing: 17.10.84

# (54) Oral anti-cancer compositions.

(30) Priority: 21.10.83 GB 8328218

(43) Date of publication of application:
10.07.85 Bulletin 85/28

(45) Publication of the grant of the patent:
03.01.90 Bulletin 90/01

(84) Designated Contracting States:
AT BE CH DE FR GB IT LI LU NL SE

(56) References cited:
WO-A-82/00825
DE-A-2 715 492
DE-A-2 845 373
FR-A-2 463 776
GB-A-1 380 228
GB-A-1 531 211
GB-A-2 060 615
US-A-3 892 790

NATIONAL TECHNICAL INFORMATION SERVICE U.S. DEPARTMENT OF COMMERCE, NTN-78/0758

The file contains technical information submitted after the application was filed and not included in this specification

(73) Proprietor: JOHNSON MATTHEY PUBLIC LIMITED COMPANY
43 Hatton Garden
London, EC1N 8EE (GB)

(72) Inventor: Barnard, Christoper Francis James
16 Luscombe Close
Caversham Reading Berkshire (GB)

(74) Representative: Arthur, Bryan Edward et al
Withers & Rogers 4 Dyer's Buildings Holborn
London EC1N 2JT (GB)

EP 0 147 926 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to orally-administrable pharmaceutical compositions for the treatment of cancer.

It is known to treat various forms of cancer or malignant tumour by administering certain coordination compounds of platinum, in particular the compound *cis*-dichloro-diammineplatinum(II), known generically as "cisplatin". Hitherto, such compounds have been administered parenterally, for example intravenously. Although parenteral administration of platinum-based drugs is beneficial for the clinician, in that dosage levels are easily determined and monitored, the availability of an orally-administrable composition would be beneficial to the patient provided that, to counteract patient-to-patient variation, the active compound was absorbed into the systemic circulation at a reasonably high level and that there was a low incidence of emesis or diarrhoea. However, despite various unsubstantiated claims in the literature to oral administrability in connection with certain such compounds, it has hitherto been found that those compounds which were investigated failed due to showing insignificant activity and in consequence it has been believed that oral administration of platinum-based drugs was not a practical proposition. For example, the compound *cis*-[diammine-(1,1-cyclobutanedicarboxylato)platinum(II)], referred to in British patent no. GB 1380228, was found to have insignificant activity when administed orally against both P388 lymphocytic leukaemia and ADJ/PC6A plasmacytoma, the therapeutic index for the latter being 2.4 compared with a value of 12.4 for the same compound against ADJ/PC6A when administered parentally.

GB—A 2060615 discloses a class of compounds of platinum (II) represented by the general formula $(A)(NH_3)Pt(X)(Y)$, in which A is a straight chain, branched chain or cyclic alkylamine and X and Y are halogen. Such compounds are said to be generally capable of being formulated so as to be suitable for parenteral or oral administration to animals affected with a malignant tumour. However, it has now been found that, whereas some compounds falling within the general scope of GB—A 2060615 do appear to show a clinical advantage from being formulated for oral administration, other compounds do not show any potential, even if they do for parenteral administration.

According to the present invention, therefore, we provide a pharmaceutical composition for the treatment of cancer by oral administration, the composition comprising a coordination compound of platinum having the general formula

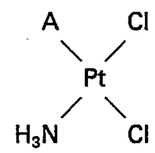

in which A is a primary amine containing a branched chain alkyl group other than a tertiary alkyl group and having from 4 to 9 carbon atoms inclusive or a cycloalkylamine having from 3 to 7 carbon atoms inclusive, with the proviso that A does not represent cyclopentylamine, in association with a pharmaceutically-acceptable carrier for oral administration.

In the general formula of compounds of platinum for use in compositions according to the invention, A may represent an alicyclic amine having the general formula cyclo-$C_zH_{2z-1}NH_2$, in which z is 3, 4, 6 or 7, preferably 4 or 6, or A may represent a primary amine containing a branched chain alkyl group other than a tertiary alkyl group and having the general formula $C_yH_{2y+1}NH_2$, in which y is an integer from 4 to 9 inclusive, preferably 4 or 5.

Pharmaceutical compositions according to the invention may be made in dosage form by combining a suitable coordination compound of platinum in an amount sufficient to produce anti-tumour activity when administered orally with a standard orally-administrable pharmaceutical carrier according to combination procedures known in the pharmaceutical art. The pharmaceutical carrier is preferably solid — that is, it provides a solid pharmaceutical composition — although liquid carriers may be used. By "solid" is meant soft capsules containing the compound formulated as a liquid or oil, as well as compressed tablets and dry filled capsules, as distinct from a syrup or suspension or other bulk liquid form.

It is preferred that compositions according to the invention contain in unit dosage form about 10 mg to 1 g of active compound.

The following substances are representative of the carriers, together with optional ingredients such as binders, lubricants and flavouring agents, which may be used for preparing pharmaceutical compositions in various forms suitable for oral administration:

edible oils such as wheat germ oil, sunflower seed oil, peanut oil — for filling soft gelatine capsules;

water-soluble vitamins such as those of the B complex or derivatives thereof, for example niacinamide and calcium pantothenate — for incorporation into dry filled capsules or compressed tablets;

lactose, glucose, magnesium sulfate, magnesium stearate and zinc sulphate — for use as binders or other excipients in the preparation of compressed tablets.

In order to produce anti-tumour activity following oral administration, it is necessary for the active ingredient of the composition — that is, the platinum coordination compound — to be absorbable to a sufficient extent into the systemic circulation. In order to establish that such absorption takes place, pharmacokinetic determinations are made, following administration, of the concentration of platinum in

the blood, urine and faeces. Determinations of the anti-tumour activity are also made.

To determine such results for selected test compounds, the compounds were administered orally via a stomach tube as a suspension in arachis oil. Dosage levels were 50 mg/kg body weight. The compounds were tested for anti-tumour activity against the ADJ/PC6A plasmacytoma grown subcutaneously in female Balb $C^-$ mice, according to the protocol as published in Chemical-Biological Interactions, vol. 11, pp. 145—161 (1975).

Experimental results are set out by way of example below. The designation of the compounds is shown in Table 1.

Absorption screening (i.e. pharmacokinetic) data for certain compounds are given in Table 2.

Anti-tumour results are quoted in Table 3.

In the absorption screening data, maximum blood levels are achieved 1 to 4 hours after administration. The excretion data indicates that the compounds are absorbed to an appreciable extent in to the systemic circulation. However, it is impossible to draw any conclusions or to predict, from the solubility data alone, the extent of absorption. Thus, while it may be thought that a high aqueous solubility would imply a high solubility in the stomach and that a high octanol solubility would imply good absorption through the stomach wall, and thus that high figures for both would be reflected by a high figure for blood levels, this is seen not to follow. For example, compound II has solubility figures in both aqueous and octanol solvents approximately five times for compound VII and yet the blood level figures are almost identical. Even more surprising is the observation that the compound with the higher solubility (compound II) required four hours to attain its peak blood level whereas compound VII, with its lower solubility, required only one hour.

In the anti-tumour tests, the $LD_{50}$ figures (the dose level causing 50% deaths) and the $ED_{90}$ figures (the dose level causing a 90% regression in tumour size) are determined by administering dosage levels ranging from lethal to non-tumour inhibitory. The therapeutic index (T.I.) is the ratio $LD_{50}/ED_{90}$ and is a measure of the selectivity of the compound as an anti-tumour agent when administered orally.

It is seen that compound I gave a TI of 7.3 against the ADJ/PC6 tumour, compound XI gave the lowest TI (4.2) and cmpound X gave a TI of 16. However, compounds VII and VIII — the even-numbered $C_4$ and $C_6$ analogues respectively of compound I — gave surprisingly high values of TI, at 14.7 and 12.7, that is, approximately twice that for the $C_5$ analogue the use of which for oral administration has already been proposed because the figures for $ED_{90}$ were low at 9.5 and 11 respectively.

TABLE 1

| Compound | Formula |
|---|---|
| I | *cis*-$[PtCl_2(NH_3)(c\text{-}C_5H_9NH_2)]$ |
| II | *cis*-$[PtCl_2(NH_3)_2]$ |
| III | *cis*-$[PtCl_2(NH_3)(n\text{-}C_3H_7NH_2)]$ |
| IV | *cis*-$[PtCl_2(NH_3)(i\text{-}C_4H_9NH_2)]$ |
| V | *cis*-$[PtCl_2(NH_3)(i\text{-}C_5H_{11}NH_2)]$ |
| VI | *cis*-$[PtCl_2(NH_3)(t\text{-}C_4H_9NH_2)]$ |
| VII | *cis*-$[PtCl_2(NH_3)(c\text{-}C_4H_7NH_2)]$ |
| VIII | *cis*-$[PtCl_2(NH_3)(c\text{-}C_6H_{11}NH_2)]$ |
| IX | *cis*-$[PtCl_2(NH_3)(i\text{-}C_4H_9NH_2)(c\text{-}C_6H_{11}NH_2)]$ |
| X | [Pt(1,2-dac)(CBDCA)] |
| XI | *cis,trans*-$[PtCl_2(OH)_2(i\text{-}C_3H_7NH_2)_2]$ |

Footnotes to Table 1:

Compound I, II, III, VI, and IX to XI are comparative examples. (Also in Tables 2 and 3).

dac represents the diaminocyclohexylamine ligand.

CBDCA represents the 1,1-cyclobutanedicarboxylate ligand.

TABLE 2

| Compound | Solubility (Pt mg/ml) | | Blood Level (Ptµg/ml) | AUC, 48h (Ptµg.h/ml) | Excretion (% dose, 48th) | |
|---|---|---|---|---|---|---|
| | AQ | OCT | | | Urine | Faeces |
| I | 1.1 | 0.07 | 1.6 (1) | 52 | 20 | 74 |
| II | 1.2 | 0.05 | 1.6 (4) | 55 | 15 | 63 |
| III | 2.0 | 0.03 | 0.6 (4) | 15 | 23 | 64 |
| IV | 0.97 | 0.08 | 0.9 (4) | 27 | 15 | 66 |
| V | 0.18 | 0.05 | 1.0 (4) | 31 | 16 | 69 |
| VI | 1.8 | 0.05 | 1.1 (2) | 34 | 24 | 65 |
| VII | 0.28 | 0.01 | 1.8 (1) | 55 | 14 | 68 |
| VIII | 0.19 | 0.06 | 0.6 (1) | 25 | 15 | 59 |
| X | 0.77 | 0.07 | 0.72 | 25 | 9 | 89 |
| XI | 8.6 | 0.16 | 1.5 | 39 | 15 | 82 |

Footnotes to Table 2:

Solubility is quoted for demineralised water (AQ) and octan-1-ol (OCT) solvents.

Blood level figures are the peak figures recorded; the figures in parentheses represent the hours, after administration, that the peak figure was attained.

AUC (Area Under Curve) figures represent the availability of platinum compound in the blood during the 48 hours following administraton of the compound and are quoted in microgram-hours of platimum per ml of blood.

TABLE 3

| Tumour | Compound | $LD_{50}$ (mg/Kg) | $ED_{90}$ (mg/Kg) | TI |
|---|---|---|---|---|
| ADJ/PC6 | I | 70 | 9.6 | 7.3 |
| | II | 140 | 24 | 5.8 |
| | III | 71 | 12 | 6 |
| | IV | 119 | 12 | 9.9 |
| | V | 142 | 21 | 6.8 |
| | VI | 71 | 12 | 5.9 |
| | VII | 140 | 9.5 | 14.7 |
| | VIII | 140 | 11 | 12.7 |
| | IX | >200 | — | — |
| | X | 1600 | 100 | 16 |
| | XI | 290 | 68.5 | 4.2 |

**Claims**

1. A pharmaceutical composition for the treatment of cancer by oral administration, the composition comprising a coordination compound of platinum having the general formula

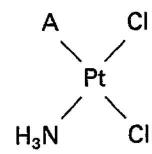

in which A is a primary amine containing a branched chain alkyl group other than a tertiary alkyl group and having from 4 to 9 carbon atoms inclusive or a cycloalkylamine having from 3 to 7 carbon atoms inclusive, with the proviso that A does not represent cyclopentylamine, in association with a pharmaceutically-acceptable carrier for oral administration.

2. A composition according to Claim 1, in which A is an alicyclic amine and is represented by the general formula cyclo-$C_zH_{2z-1}NH_2$, in which z is 3, 4, 6 or 7.

3. A composition according to Claim 1, in which A is a primary amine containing a branched chain alkyl group other than a tertiary alkyl group and is represented by the general formula $C_yH_{2y+1}NH_2$, in which y is an integer from 4 to 9 inclusive.

4. A composition according to Claim 2, in which z is 4 or 6.

5. A composition according to Claim 3, in which y is 4 or 5.

**Patentansprüche**

1. Pharmazeutische Zusammensetzung für die Behandlung von Krebs durch orale Verabreichung, dadurch gekennzeichnet, daß diese Zusammensetzung eine Koordinatonsverbindung von Platin, welche die allgemeine Formel

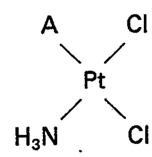

hat, worin A ein eine verzweigte Alkylgruppe mit 4 bis einschließlich 9 Kohlenstoffatomen, jedoch keine tertiäre Alkylgruppe, enthaltendes primäres Amn oder ein Cycloalkylamin mit 3 bis einschließlich 7 Kohlenstoffatomen, jedoch kein Cyclopentylamin, bedeutet, als Wirkstoff in Verbindung mit einem pharmazeutisch annehmbaren Träger für eine orale Verabreichung enthält.

2. Zusammensetzung nach Anspruch 1, dadurch gekennzeichnet, daß A ein Cycloalkylamin der allgemeinen Formel Cyclo-$C_zH_{2z-1}NH_2$, worin z für 3, 4, 6 oder 7 steht, bedeutet.

3. Zusammensetzung nach Anspruch 1, dadurch gekennzeichnet, daß A ein eine verzweigte Alkylgrupe, jedoch keine tertiäre Alkylgruppe, enthaltendes primäres Amin der allgemeinen Formel $C_yH_{2y-1}NH_2$, worin y für eine Zahl von 4 bis einschließlich 9 steht, bedeutet.

4. Zusammensetzung nach Anspruch 2, dadurch gekennzeichnet, daß z für 4 oder 6 steht.

5. Zusammensetzung nach Anspruch 3, dadurch gekennzeichnet, daß y für 4 oder 5 steht.

**Revendications**

1. Une composition pharmaceutique pour le traitement du cancer par administration par voie orale, la composition comprenant un composé de coordination de platine ayant la formule générale:

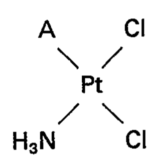

dans laquelle A est une amine primaire contenant un groupement alkyle à chaîne ramifiée autre qu'un groupement alkyle tertiaire et ayant de 4 à 9 atomes de carbone inclusivement ou une cycloalkylamine ayant de 3 à 7 atomes de carbone inclusivement, avec la condition que A ne représente pas une cyclopentylamine, en association avec un véhicule pharmaceutiquement acceptable pour une administration par voie orale.

2. Une composition selon la revendication 1 dans laquelle A est une amine alicyclique et est représentée par la formule générale cyclo-$C_zH_{2z-1}NH_2$, où z vaut 3, 4, 6 ou 7.

3. Une composition selon la revendication 1, dans laquelle A est une amine primaire contenant un groupement alkyle à chaîne ramifiée autre qu'un groupement alkyle tertiaire et est représenté par la formule générale $C_yH_{2y+1}NH_2$, où y est un nombre entier de 4 à 9 inclusivement.

4. Une composition selon la revendication 2, dans laquelle z vaut 4 ou 6.

5. Une composition selon la revendication 3, dans laquelle y vaut 4 ou 5.